**Europäisches Patentamt**

**European Patent Office**

**Office européen des brevets**

(11) Veröffentlichungsnummer: **0 290 811 B1**

(12) **EUROPÄISCHE PATENTSCHRIFT**

(45) Veröffentlichungstag der Patentschrift: **10.06.92**

(51) Int. Cl.⁵: **G01C 17/30**, G01R 33/02, G01V 3/40, G01R 33/06

(21) Anmeldenummer: **88106011.5**

(22) Anmeldetag: **15.04.88**

(54) **Vorrichtung zur Erfassung von Stärke und Richtung eines Magnetfeldes, insbesondere des Erdmagnetfeldes.**

(30) Priorität: **11.05.87 CH 1777/87**

(43) Veröffentlichungstag der Anmeldung:
**17.11.88 Patentblatt 88/46**

(45) Bekanntmachung des Hinweises auf die Patenterteilung:
**10.06.92 Patentblatt 92/24**

(84) Benannte Vertragsstaaten:
**AT CH FR GB IT LI NL SE**

(56) Entgegenhaltungen:
EP-A- 0 221 436
DE-A- 1 589 504
DE-A- 1 956 001
DE-A- 3 235 751
US-A- 3 873 913

(73) Patentinhaber: **Leica Heerbrugg AG**

**CH-9435 Heerbrugg(CH)**

(72) Erfinder: **Brun, Robert, Dr.**
**Rebengasse 12**
**CH-9436 Balgach(CH)**
Erfinder: **Ramseier, Ernst, Dr.**
**Auerstrasse 13**
**CH-9435 Heerbrugg(CH)**

(74) Vertreter: **EGLI-EUROPEAN PATENT ATTORNEYS**
**Horneggstrasse 4**
**CH-8008 Zürich(CH)**

**Beschreibung**

Die Erfindung betrifft eine Vorrichtung zur Erfassung von Stärke und Richtung eines Magnetfeldes gemäss dem Oberbegriff des Patentanspruchs 1, sowie eine Verwendung dieser Vorrichtung.

Eine Vorrichtung der genannten Art ist beispielsweise aus Seiten 8 bis 10 der Broschüre "Anwendungen der Magnetfeldsensoren KMZ 10", Technische Information Philips-VALVO 861105 (1986), 17 Seiten, bekannt: danach kann mit zwei in einer Spule rechtwinklig zueinander angeordneten Sensoren ein Kompass hergestellt werden, wobei sich beide Sensoren mit einer Spule umschalten lassen.

Wenn nämlich ein magnetoresistiver Sensor zur Erfassung von Stärke und Richtung eines Magnetfeldes verwendet wird, ist es nötig, am Sensor mittels eines darauf einwirkenden magnetischen Hilfsfeldes eine Vorzugsrichtung zu definieren. Das Hilfsfeld dient dazu, das regellose Umklappen der Weiss'schen Bezirke unter dem Einfluss veränderlicher Magnetfelder zu verhindern, oder es bewirkt zumindest die Wiederherstellung eines definierten Magnetisierungszustandes des Sensors nach dem Abklingen einer darauf einwirkenden starken magnetischen Störung.

Es ist nun aus der zitierten Broschüre bekannt, das magnetische Hilfsfeld durch eine stromdurchflossene Spule zu erzeugen. Ferner ist bekannt, eine von einem getakteten Strom durchflossene Spule zu verwenden, wenn ein schwaches Feld wie beispielsweise das Erdmagnetfeld zu erfassen ist, wobei das Hilfsfeld zum Zeitpunkt der Erfassung ausgeschaltet wird, um zu vermeiden, dass die Empfindlichkeit des Sensors durch das Hilfsfeld vermindert wird. Ausserdem ist bekannt, durch systematisches Ummagnetisieren des Sensors die Auswirkung der stark temperaturabhängigen und vom einen zum anderen Exemplar des Sensors herstellungsbedingt variablen Nullwertverschiebung ("DC offset") zu vermeiden.

Eine vollständige Erfassung der Stärke und Richtung des Erdmagnetfeldes in einem präzisen elektronischen Kompass erfordert aber drei Sensoren mit zueinander nicht koplanaren Messrichtungen (zur Bestimmung der Horizontalebene sind ausserdem Neigungs- oder Schwerkraftsensoren erforderlich). Zu diesem Problem gibt die zitierte Broschüre folgenden Hinweis: "Für Messungen auch in der dritten Richtung (senkrecht zur XY-Ebene) muss allerdings eine zweite Spule verwendet werden. Es hat sich nämlich gezeigt, dass Anordnungen mit nur einer Spule für drei Richtungen (und damit für drei Sensoren) keine befriedigenden Lösungen ergeben."

Bei Verwendung mehrerer gerader Spulen (Solenoide), wie es in der zitierten Broschüre vorgeschlagen wird, ist nämlich nachteilig, dass die Anordnung der Spulen zueinander gewissen Einschränkungen unterworfen ist: so ist es beispielsweise nötig, einen grösseren räumlichen Abstand zwischen den Spulen vorzusehen oder das Wechselfeld jeder Spule abzuschirmen, um eine gegenseitige Beeinflussung zu vermeiden.

Ein weiterer Nachteil der Verwendung offener Spulen ist, dass jede an ihren Enden das Hilfsfeld frei abstrahlt, was zu Problemen der elektromagnetischen Verträglichkeit und zu Verlusten besonders bei Hochfrequenzabschirmung, d.h. zu einem erhöhten Leistungsbedarf der Speisung der Vorrichtung führt.

Dennoch ist es wünschenswert, die drei Komponenten des Magnetfeldes im dreidimensionalen Raum gleichzeitig zu messen, um Messfehler auszuschliessen, die bei schnell bewegter Messstelle (z.B. auf einem Fahrzeug) oder sich schnell änderndem Magnetfeld auftreten, wenn die Messung der verschiedenen Komponenten nacheinander oder die Messung einer der Komponenten in bezug auf die beiden anderen mit deutlichem Zeitverzug erfolgt.

Aufgabe der Erfindung ist es, zur gleichzeitigen Erfassung der drei Komponenten des Magnetfeldes im dreidimensionalen Raum mindestens drei Sensoren gleichzeitig mit dem gleichen wohldefinierten Hilfsfeld zu beaufschlagen, ohne dabei die erwähnten Nachteile der Verwendung von mehr als einer Spule in Kauf zu nehmen.

Diese Aufgabe wird erfindungsgemäss gelöst durch eine Vorrichtung mit der im Patentanspruch 1 angegebenen Kombination von Merkmalen. Vorteilhafte Weiterbildungen der erfindungsgemässen Vorrichtung und eine Verwendung davon ergeben sich aus den entsprechenden abhängigen Ansprüchen bzw. aus Anspruch 11 und abhängigen Anspruch 12.

Mit der erfindungsgemässen Vorrichtung ist es möglich, die drei Komponenten des Magnetfeldes im dreidimensionalen Raum nicht nur zur gleichen Zeit, sondern auch praktisch am gleichen Ort zu messen, weil die Abmessungen der Vorrichtung wesentlich kleiner gehalten werden können, als wenn man die bekannte Vorrichtung mit drei Sensoren versieht. Zusätzlich wird erreicht, dass die Vorrichtung nicht in einer vorgegebenen räumlichen Lage (d.h. im vorhinein horizontiert) betrieben werden muss. Diese Möglichkeiten ergeben bedeutsame Vorteile beim Einsatz der Vorrichtung als elektronischer Kompass in schnell bewegten Luft-, See- oder Landfahrzeugen.

Bei der erfindungsgemässen Form der das Hilfsfeld erzeugenden Spule ist das Hilfsfeld im Inneren der Spule im wesentlichen homogen, und zwar auch in den kleinen Aussparungen zur Aufnahme der Sensoren und deren Anschlüsse: für den Einsatz der Vorrichtung als elektronischer Kompass ist dieses Feld genügend homogen. Dazu kommt, dass die Feldstärke des Hilfsfeldes im Inneren einer toroidalen Spule leicht zu berechnen ist.

Um die Homogenität des Hilfsfeldes zu verbessern und mehr Platz zur Aufnahme der Sensoren verfügbar zu machen, wobei gewährleistet bleibt, dass alle Sensoren vom Hilfsfeld mit gleicher Stärke beaufschlagt werden, kann der Körper eine Form aufweisen, die von einem Torus abgeleitet ist, indem die gedachte Mittellinie des Torus, stets in derselben Ebene bleibend, zu einer geschlossenen Linie mit Abschnitten verschiedener Krümmung verformt ist. Beispielsweise kann die Mittellinie im Bereich der Sensoren gerade Abschnitte und kreisbogenförmige Abschnitte aufweisen, die aneinander wechselweise anschliessend miteinander verbunden sind.

Des weiteren ist insbesondere für den Einsatz der Vorrichtung als elektronischer Kompass in schnell bewegten Luft-, See-oder Landfahrzeugen vorteilhaft, dass die Bauweise der Vorrichtung kompakt sein kann und die gegenseitige Lage der Sensoren unverrückbar ist.

Gleich wie bei der bekannten, eingangs zitierten Vorrichtung ist es bei der erfindungsgemässen Vorrichtung möglich, durch systematisches Ummagnetisieren der Sensoren die Auswirkung der temperaturabhängigen und herstellungbedingten Nullwertverschiebung ("DC" offset) zu vermeiden.

Schliesslich werden die vorangehend erwähnten, mit dem Vorhandensein von Spulenenden verbundenen Nachteile, nämlich die Probleme mit der elektromagnetischen Verträglichkeit und den Leistungsverlusten, mit der erfindungsgemässen Vorrichtung vermieden.

Es wird hier noch auf folgende Literaturstellen verwiesen, die aus Permalloy und in Dünnschichttechnik hergestellte magnetoresistive Sensoren und deren Verwendung in der Datentechnik, Messtechnik und Navigation betreffen: "Magnetoresistive Sensoren", Technische Information Philips-VALVO 840323 (1984), 8 Seiten; "The permalloy magnetoresistive sensor -properties and applications", W.Kwiatowski und S.Tumanski, J. Phys E. Sci. Instrum. 19 (1986) 502-515; "Magnetoresistive permalloy sensors and magnetometers", W.Kwiatowski und S.Tumanski, Archiwum Elektrotechniki 32 (1983) 55-64.

Zum technologischen Hintergrund bezüglich des Prinzips und der Verwendung von magnetoresistiven Sensoren wird auf folgende Literaturstellen verwiesen: "The magnetoresistive sensor - a sensitive device for detecting magnetic field variation", U.Dibbern und A.Petersen, Electronic Components and Applications, 5/3 (1983) 148-153; "Drehwinkelmessung mit Magnetfeldsensoren", G.Reiniger, Elektronik 23 (1986) 129-136; "Magnetoresistive response of small permalloy features", S. K. Decker und C. Tsang, IEEE Trans. on Magnetics, Mag-16/5 (1980) 643-645; "Magnetoresistance in laminated NiFe films", J.A.C.Van Oyen et al., J. Appl. Phys. 53/3 (1982) 2596-2598.

Im nachfolgenden werden Beispiele von Ausbildungen der erfindungsgemässen Vorrichtung unter Bezugnahme auf die Zeichnungen näher erläutert. Es zeigen:

Fig. 1    eine schematische Ansicht eines ersten Beispiels einer Ausbildung der erfindungsgemässen Vorrichtung im Schnitt durch ihre mittlere Ebene,

Fig. 2    eine schematische Ansicht der Ausbildung der Fig. 1 im Schnitt durch die in Fig. 1 mit der Linie A-A bezeichnete Ebene, und

Fig. 3    eine schematische Ansicht eines zweiten Beispiels einer Ausbildung der erfindungsgemässen Vorrichtung im Schnitt durch ihre mittlere Ebene.

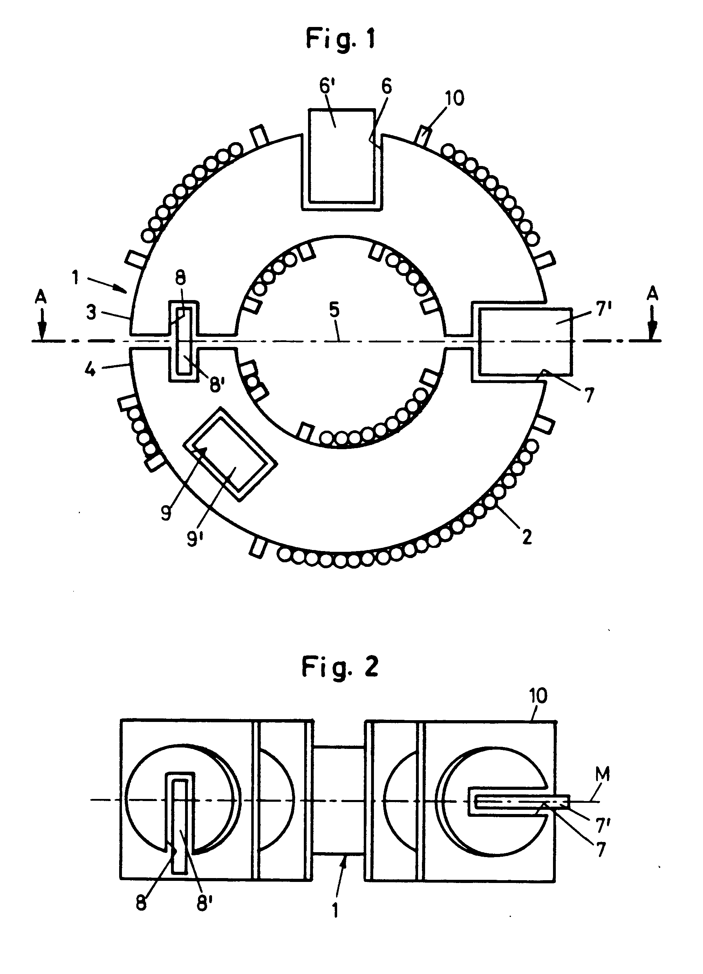

In Fig. 1 und 2, welche die Erfindung in einer schematischen Dasstellungsweise erläutern, ist eine erste Ausbildung der erfindungsgemässen Vorrichtung mit einem im wesentlichen toroidalen Körper 1 dargestellt. Das Material dieses Körpers kann ein beliebiges nichtmagnetisches isolierendes Material, beispielsweise ein Kunststoff oder auch Keramik sein. Bei Verwendung von Kunststoff wird als Material des Körpers 1 vorzugsweise ein glasfaserverstärkter Kunststoff bekannter Art wie ein glasfaserverstärktes Epoxyharz verwendet.

In Fig. 1 ist der Körper 1 im Schnitt durch seine mittlere Ebene M (die Ebene der kreisförmigen Mittellinie des Torus), in Fig. 2 im diametralen Schnitt durch die in Fig. 1 mit der Linie A-A bezeichnete Ebene dargestellt. Der Körper 1 bildet den Kern einer toroidalen Spule 2, die im wesentlichen, d.h. mit den nachstehend erläuterten Ausnahmen, auf dem Körper 1 in regelmässigen Windungen aufgewickelt ist. In Fig. 1 sind von der Spule 2 nur einige Windungen schematisch (insbesondere überproportional dimensioniert) im Querschnitt dargestellt worden, während in Fig. 2 die Windungen, die darin sichtbar sein sollten, überhaupt nicht eingezeichnet worden sind, da eine Windung im richtigen Massstab in Fig. 2 kaum erkennbar wäre und eine überdimensionierte Darstellung einer einzelnen Windung oder eine richtige Darstellung der ganzen Spule das Verständnis der Fig. 2 beeinträchtigen würde.

Um die Herstellung der Wicklung und die Montage der Vorrichtung zu erleichtern, besteht der Körper 1 aus zwei Teilen 3 und 4. Vorzugsweise sind diese Teile 3 und 4 von gleicher Grösse, so dass sie sich entlang einer diametralen Ebene 5 des Körpers 1 zusammenfügen. Der toroidale Körper 1 kann aber bei Bedarf auch aus mehr als zwei Teilen bestehen. Die Befestigung der beiden Teile 3 und 4 aufeinander ist nicht dargestellt, sie kann beispielsweise durch Verklebung und/oder Befestigung auf einer gemeinsamen Unterlage erfolgen. In Fig. 1 sind die beiden Teile 3 und 4 mit einem kleinen dazwischenliegenden Abstand dargestellt, dies ist jedoch nur eine zeichnerische Massnahme zur Klarheit der Darstellung, die beiden Teile 3 und 4 liegen in Wirklichkeit aneinander an.

Der Körper 1 ist mit drei Ausnehmungen 6, 7, 8 versehen, die zur Aufnahme je eines magnetoresistiven Sensors 6', 7', 8' vorgesehen sind. Solche magnetoresistive Sensoren sind im wesentlichen rechteckige dünne Plättchen, die je eine Messrichtung aufweisen, in der sie aufgrund ihrer magnetoresistiven Eigenschaften auf die Feldstärke eines daran angelegten Magnetfeldes empfindlich sind: die Komponente dieser Feldstärke in der Messrichtung des Sensors wird von diesem in ein elektrisches Signal umgesetzt. In Fig. 1 und 2 sind die Sensoren 6', 7', 8' mit einem deutlichen Abstand zur Wandung der zugeordneten Ausnehmung 6, 7, 8 dargestellt, dies ist jedoch nur eine zeichnerische Massnahme zur Klarheit der Darstellung: die Sensoren liegen in Wirklichkeit satt in den Ausnehmungen, damit ihre Lage genau bestimmt ist, und sie sind darin auf beliebige Weise unverrückbar befestigt, beispielsweise eingeklebt. Der Klebstoff dient übrigens auch der Kühlung der Sensoren, indem er die in den Sensoren elektrisch entwickelte Wärme in den Körper 1 ableitet.

Im Bereich der Sensoren 6', 7', 8' kann die Wicklung der Spule 2 nicht genau so regelmässig wie im Bereich zwischen den Sensoren weitergeführt werden, d.h. der jeweilige Bereich einer Ausnehmung und des zugeordneten Sensors wird auf geeignete Weise übersprungen, beispielsweise durch Aufteilen der Wicklung oder Änderung des Wicklungsschrittes. Diese Ausnahmen zur Regelmässigkeit der Wicklung sind jedoch nicht erheblich, so dass die gesamte Vorrichtung auch mit den Ausnehmungen und den darin liegenden Sensoren im wesentlichen toroidal ist.

Die Zweiteilung des Körpers 1 erleichtert das Aufbringen der Wicklungen der Spule 2 ganz wesentlich. Hilfsmittel wie Stege, Scheiben oder Platten 10 beispielsweise aus Kunststoff können darauf angeordnet oder als integrale Teile des Spulenkörpers gefertigt werden und zur Befestigung des gesamten toroidalen Gebildes auf eine Unterlage dienen.

Im Körper 1 sind die Ausnehmungen derart orientiert, dass die Messrichtungen 6', 7', 8' der drei Sensoren zueinander orthogonal liegen. Somit werden drei zueinander orthogonal liegende Komponenten der zu messenden Feldstärke erfasst, was die Bestimmung von Stärke und Richtung des zu messenden Magnetfeldes erlaubt.

Zur Erhöhung der Genauigkeit und Sicherheit des Messergebnisses kann am Körper 1 mindestens ein weiterer Sensor 9' zusätzlich zu den Sensoren 6', 7', 8' in einer jeweils entsprechenden Ausnehmung angeordnet werden. Die Winkellage der zusätzlichen Sensoren ist dabei entsprechend dem verfolgten Zweck gewählt. In dem in Fig. 1 und 2 dargestellten Beispiel ist der Körper 1 mit einer vierten Ausnehmung 9 versehen, die zur Aufnahme eines vierten magnetoresistiven Sensors 9' vorgesehen ist. Die Messrichtung dieses Sensors 9' ist grundsätzlich von den Messrichtungen der Sensoren 6', 7, 8' verschieden. Damit der Sensor 9' seinen Zweck auf gleiche Weise im Zusammenwirkung mit jedem der drei anderen Sensoren 6', 7' und 8' erfüllen kann, ist er vorzugsweise so angeordnet, dass seine Messrichtung zu jeder der drei anderen, zueinander orthogonalen Messrichtungen der Sensoren 6', 7, 8' im gleichen Winkel liegt.In anderen Worten, wenn man sich vorstellt, dass die Messrichtungen der Sensoren 6', 7' und 8' die Kanten eines Würfels bilden, so liegt die Messrichtung des Sensors 9' auf einer der Raumdiagonalen dieses Würfels. Der in diesem Beispiel mit dem Sensor 9', im allgemeinen Fall mit anderen Sensoren erfasste Messwert ist dazu bestimmt, mit dem von den drei anderen Sensoren 6', 7' und 8' erfassten Messwerten kombiniert zu werden, um, wie beispielsweise vorstehend erwähnt, die Genauigkeit und Sicherheit des Messergebnisses zu erhöhen.

Die Spule 2 dient zum Erzeugen eines an die Sensoren 6', 7', 8' und 9' angelegten magnetischen Hilfsfeldes. Der Zweck dieses Hilfsfeldes ist an sich bekannt und eingangs erläutert worden.

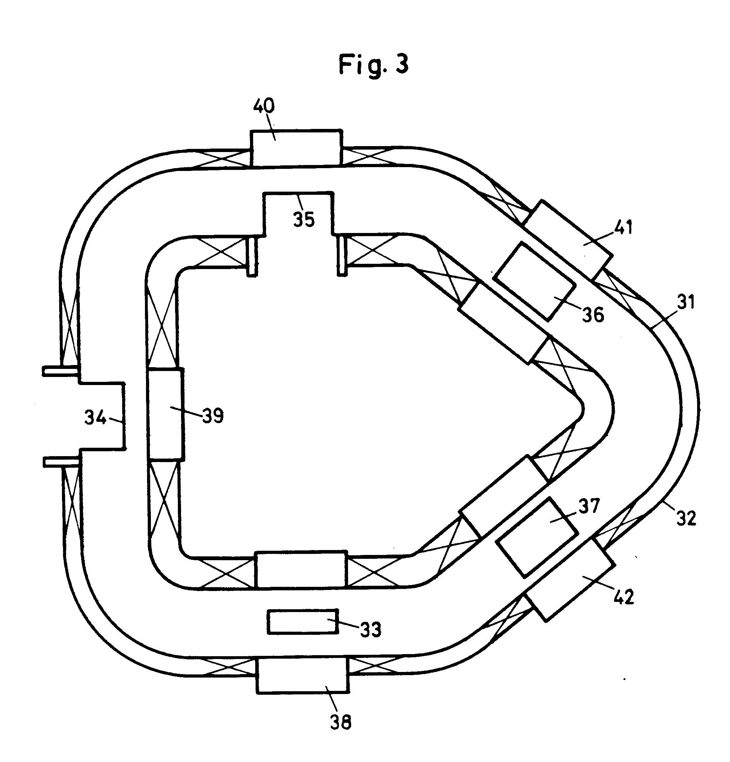

In Fig. 3, welche die Erfindung ebenfalls in einer schematischen Darstellungsweise erläutert, ist eine zweite Ausbildung der erfindungsgemässen Vorrichtung mit einem Körper 31 von anderer Form als in Fig. 1 dargestellt. In dieser Ausbildung ist die Form des Körpers 31 von der Form eines Torus durch Verformung der gedachten Mittellinie des Torus abgeleitet, wobei diese gedachte Mittellinie stets in derselben Ebene bleibt, weiterhin eine geschlossene Linie bildet, und nun Abschnitte verschiedener Krümmung aufweist (bei einer Krümmung gleich Null sind die betreffenden Abschnitte geradlinig).Der Körper 31 weist daher eine einem Torus topologisch äquivalente Form auf, die in Kurzform als deformierter Ring bezeichnet werden kann. Näheres wird weiter unten erläutert. Das Material des Körpers 30 ist im Zusammenhang mit dem Körper 1 bereits beschrieben worden. Der Körper 31 kann aus zwei oder mehreren Teilen bestehen, wie es im Zusammenhang mit Fig. 1 beschrieben wurde.

In Fig. 3 ist der Körper 31 analog zur Darstellungsweise der Fig. 1 im Schnitt durch seine mittlere Ebene dargestellt. Der Körper 31 bildet den Kern einer Spule 32, die im wesentlichen, d.h. mit den im Zusammenhang mit Fig. 1 erläuterten Ausnahmen, auf dem Körper 31 in regelmässigen Windungen aufgewickelt ist. In Fig. 3 ist diese Spule 32 schematisch im Schnitt dargestellt.

Der Körper 1 ist mit fünf Ausnehmungen 33, 34, 35, 36 und 37 versehen, die zur Aufnahme je eines nicht dargestellten magnetoresistiven Sensors der bereits im Zusammenhang mit Fig. 1 angegebenen Art vorgesehen sind. Im Bereich der Ausnehmungen 33, 34, 35, 36 und 37 ist die gedachte Mittellinie des Körpers 31 geradlinig, d.h. jede Ausnehmung befindet sich in einem von fünf im wesentlichen zylindrischen Abschnitten des Körpers 31. Zwischen diesen zylindrischen Abschnitten ist die gedachte Mittellinie des Körpers 31 gekrümmt. Entlang der gedachten Mittellinie sind also wechselweise fünf geradlinige und fünf daran tangential anschliessende, im wesentlichen kreisbogenförmige Abschnitte zu einer geschlossenen ungebrochenen Kurve zusammengeschlossen. Der um diese gedachte Mittellinie konstruierte Körper 31 weist einen im wesentlichen kreisförmigen Querschnitt auf und ist daher, bildlich ausgedrückt, ein deformierter Torus.

Um die Festigkeit des Körpers 1 bzw. 31 im Bereich der Ausnehmungen 6, 7, 8 und 9 bzw. 33, 34, 35, 36 und 37 zu erhöhen und um zur Mittelebene fest verbundene Auflageflächen zu erhalten, können statt der Platten 10 quaderförmige Verstärkungen 38, 39, 40, 41 und 42 angebracht werden.

Wie die Spule 2 in Fig. 1 ist die Spule 32 im Bereich der Ausnehmungen 33, 34, 35, 36 und 37 unterbrochen, d.h. der jeweilige Bereich einer Ausnehmung und des zugeordneten Sensors wird auf geeignete Weise übersprungen, beispielsweise durch Aufteilen der Wicklung oder Änderung des Wicklungsschrittes. Wiederum sind diese Ausnahmen zur Regelmässigkeit der Wicklung nicht erheblich.

Im Körper 31 sind die Ausnehmungen 33, 34, 35, 36 und 37 verschiedentlich orientiert, um den fünf Sensoren verschiedene Messrichtungen zu geben und somit die Genauigkeit und Sicherheit des Messergebnisses zu erhöhen, wie bereits im Zusammenhang mit Fig. 1 beschrieben wurde.

In der Ausbildung nach Fig. 3 können beispielsweise in den drei Ausnehmungen 33, 34, 35 jeweilige Sensoren mit zueinander orthogonalen Messrichtungen von der Art der Sensoren 6', 7, 8' in Fig. 1 und in den zwei Ausnehmungen 36, 37 jeweilige zusätzliche Sensoren von der Art des Sensors 9' in Fig. 1 angeordnet werden. Diese fünf Sensoren werden gemeinsam vom Magnetfeld der gemeinsamen Spule 32 vorgespannt.

Die beschriebene und in Fig. 1 bis 3 in verschiedenen Ausbildungen dargestellte Vorrichtung ist ganz besonders zur Erfassung der Stärke und Richtung des Erdmagnetfeldes geeignet, beispielsweise zur Verwendung in einer Einrichtung zur magnetischen Nordfindung in einem Luft-, See- oder Landfahrzeug. Da alle drei Komponenten des Erdmagnetfeldes erfasst werden, ist die Einbaulage des Körpers 1 in das Fahrzeug bzw. im Kompass des Fahrzeugs beliebig. Allerdings wird die Genauigkeit der Nordfindung erhöht, wenn eine Lage gewählt wird, bei welcher keine der drei Komponenten ständig dominiert. Bei der bevorzugten Lage ist der Körper 1 in der Einrichtung zur magnetischen Nordfindung so eingebaut, dass die mittlere Ebene M des Körpers 1 mit der Horizontalebene einen Winkel bildet. Dieser Winkel wird so bestimmt, dass bei nach Norden weisender Vorrichtung mindestens zwei Sensoren gleich grosse Signale abgeben; er beträgt daher in mittleren geographischen Breiten etwa 20°.

Selbstverständlich eignet sich die in Fig. 1 bis 3 dargestellte Vorrichtung auch zur Erfassung der Stärke und Richtung von anderen Magnetfeldern als dem Erdmagnetfeld, wenn deren Feldstärke in derselben Grössenordnung wie das Erdmagnetfeld liegt oder davon um bis zu zwei Grössenordnungen abweicht, also zwischen $10^{-2}$ und $10^{2}$ mal derjenigen des Erdmagnetfeldes liegt.

In einem konkreten Beispiel einer solchen Vorrichtung werden magnetoresistive Sensoren von einem im Handel erhältlichen Typ mit einer viereckigen empfindlichen Fläche von etwa 1,6 mm Seitenlänge verwendet. Die Sensoren sind in Kunststoff eingebettet und präsentieren sich als etwa viereckiges Plättchen von etwa 5 mm Seitenlänge und etwa 1,8 mm Höhe (nähere Angaben sind in der eingangs zitierten Druckschrift enthalten). Das magnetische Hilfsfeld muss an diesen Sensoren etwa 3 kA/m betragen. Als Bedingung für die Homogenität des Hilfsfeldes über den gesamten magnetisch empfindlichen Bereich jeden Sensors kann festgelegt werden, dass die Querkomponenten des Hilfsfeldes, die sich aus der Krümmung seiner Feldlinien im toroidalen oder einemTorus analogen Körper ergeben, an keiner Stelle des empfindlichen Bereichs grösser sein dürfen als ein vorbestimmter Bruchteil der Längskomponente des Hilfsfeldes auf der Mittellinie des Körpers. Aus den Dimensionen des magnetisch empfindlichen Bereichs der Sensoren sowie dem als höchstzulässig gewählten Verhältnis der Quer- zur Längskomponente, beispielsweise 10 %, lässt sich der kleinstzulässige Radius an der betreffenden Stelle des Körpers mit einfachen geometrischen und trigonometrischen Überlegungen berechnen.

**Patentansprüche**

1. Vorrichtung zur Erfassung von Stärke und Richtung eines Magnetfeldes, umfassend eine Mehrzahl von magnetoresistiven Sensoren, denen je eine verschiedene Messrichtung zugeordnet ist, und eine Spule zum Erzeugen eines an die Sensoren angelegten magnetischen Hilfsfeldes,
gekennzeichnet durch
einen Körper (1;31) aus nichtmagnetischem isolierendem Material und von einer einem Torus topologisch äquivalenten Form, auf dem die Spule (2;32) in im wesentlichen regelmässigen Windungen aufgewickelt ist und der mit mindestens drei Ausnehmungen (6,7,8;33,34,35) zur Aufnahme je eines Sensors (6′,7′,8′) versehen ist.

2. Vorrichtung nach Anspruch 1, dadurch gekennzeichnet, dass die Messrichtungen von drei Sensoren (6′,7′,8′) zueinander orthogonal angeordnet sind.

3. Vorrichtung nach Anspruch 1, dadurch gekennzeichnet, dass der Körper (1;31) aus glasfaserverstärktem Kunststoff besteht.

4. Vorrichtung nach Anspruch 1, dadurch gekennzeichnet, dass der Körper (1;31) aus Keramik besteht.

5. Vorrichtung nach Anspruch 1, dadurch gekennzeichnet, dass der Körper (1) im wesentlichen die Form eines Torus hat.

6. Vorrichtung nach Anspruch 1, dadurch gekennzeichnet, dass der Körper (31) eine Form hat, die von einem Torus abgeleitet ist, indem die gedachte Mittellinie des Torus, stets in derselben Ebene bleibend, zu einer geschlossenen Linie mit Abschnitten verschiedener Krümmung verformt ist.

7. Vorrichtung nach Anspruch 1, dadurch gekennzeichnet, dass der Kürper (1;31) mit mindestens einer weiteren Ausnehmung (9;36,37) zur Aufnahme eines weiteren Sensors (9′) versehen ist, dessen Messrichtung von den Messrichtungen der genannten anderen drei Sensoren (6′,7′,8′) verschieden ist.

8. Vorrichtung nach Anspruch 2, dadurch gekennzeichnet, dass der Körper (1) mit einer weiteren Ausnehmung (9) zur Aufnahme eines weiteren Sensors (9′) versehen ist, dessen Messrichtung zu jeder der Messrichtungen der genannten drei zueinander orthogonalen Sensoren (6′,7′,8′) so im gleichen Winkel liegt wie die Raumdiagonale eines Würfels zu den Kanten desselben.

9. Vorrichtung nach Anspruch 1, dadurch gekennzeichnet, dass der Körper (1;31) mehrteilig ausgebildet ist.

10. Vorrichtung nach den Ansprüchen 5 und 9 oder nach den Ansprüchen 6 und 9, dadurch gekennzeichnet, dass der Körper (1) zweiteilig ausgebildet ist und die Zusammenfügung seiner beiden Teile (3,4) entlang einer diametralen Symmetrieebene (5) des Körpers (1) erfolgt.

11. Verwendung der Vorrichtung nach Anspruch 1 zur Erfassung der Stärke und Richtung eines Magnetfeldes, dessen Feldstärke in der Grössenordnung des Erdmagnetfeldes oder bis zu zwei Grössenordnungen darüber oder darunter liegt.

12. Verwendung nach Anspruch 11 in einer Einrichtung zur magnetischen Nordfindung, dadurch gekennzeichnet, dass die mittlere Ebene (M) des Körpers (1;31) in einem vom geographischen Breitengrad des Einsatzortes der Vorrichtung abhängigen, in mittleren Breitengraden etwa 20° betragenden Winkel zur Horizontalebene angeordnet ist.

**Claims**

1. A device for the acquisition of the strength and direction of a magnetic field, comprising a multiplicity of magneto-resistant sensors, each of which is assigned a different direction of measurement, and a spool for generating an auxiliary magnetic field applied to the sensors,
characterized by
a body (1;31) made of non-magnetic insulating material and having a shape topologically equivalent to a torus, on which the spool (2;32) is wound in essentially regular windings and which is provided with at least three recesses (6,7,8;33,34,35) each to accommodate a sensor (6',7',8').

2. A device according to claim 1, characterized in that the directions of measurement of three sensors (6',7',8') are arranged orthogonal to each other.

3. A device according to claim 1, characterized in that the body (1;31) is made of glass fibre reinforced synthetic material.

4. A device according to claim 1, characterized in that the body (1;31) is made of ceramic.

5.  A device according to claim 1, characterized in that the body (1) has essentially the shape of a torus.

6.  A device according to claim 1, characterized in that the body (31) has a shape derived from a torus in that the ideal median line of the torus is deformed to a closed line having sections of different curvature while constantly remaining in one and the same plane.

7.  A device according to claim 1, characterized in that the body (1;31) is provided with at least one other recess (9;36,37) to accommodate still another sensor (9') whose direction of measurement differs from the direction of measurement of said three other sensors (6',7',8').

8.  A device according to claim 2, characterized in that the body (1) is provided with another recess (9) to accommodate still another sensor (9') whose direction of measurement is oriented at equal angles in respect of each one direction of measurement of said three mutually orthogonal sensors (6',7',8') in the same manner as the spatial diagonal of a cube is oriented to the edges of the latter.

9.  A device according to claim 1, characterized in that the body (1;31) is constructed with several parts.

10. A device according to claims 5 and 9 or according to claims 6 and 9, characterized in that the body (1) is constructed with two parts and the joining together of its two parts (3,4) takes place along a diametrical plane of symmetry (5) of the body (1).

11. A use of the device according to claim 1 for the acquisition of the strength and direction of a magnetic field whose field strength is of the order of magnitude of the earth magnetic field or above or below the latter by at most two orders of magnitude.

12. Use according to claim 11 in an appliance for finding the magnetic north, characterized in that the median plane (M) of the body (1;31) is arranged with respect to the horizontal plane at an angle which depends on the geographical latitude of the location of operation of the device and has a value of about 20° in middle latitudes.

**Revendications**

1.  Dispositif pour la saisie de la grandeur et de la direction d'un champ magnétique, comprenant une multiplicité de capteurs à magnétorésistance à chacun desquels est attribuée une direction de mesure différente, ainsi qu'une bobine destinée à produire un champ magnétique auxiliaire appliqué aux capteurs, caractérisé par
    un corps (1;31) en matériau isolant non magnétique et qui présente une forme topologiquement équivalente à un tore, sur lequel la bobine (2;32) est bobinée en tours essentiellement réguliers et qui est pourvu d'au moins trois cavités (6,7,8;33,34,35) destinées chacune à recevoir un capteur (6',7',8').

2.  Dispositif selon la revendication 1, caractérisé en ce que les directions de mesure de trois capteurs (6',7',8') sont disposées orthogonalement les unes par rapport aux autres.

3.  Dispositif selon la revendication 1, caractérisé en ce que le corps (1;31) est fait de matière synthétique renforcée de fibre de verre.

4.  Dispositif selon la revendication 1, caractérisé en ce que le corps (1;31) est fait de céramique.

5.  Dispositif selon la revendication 1, caractérisé en ce que le corps (1) présente essentiellement la forme d'un tore.

6.  Dispositif selon la revendication 1, caractérisé en ce que le corps (31) présente une forme qui est dérivée d'un tore en ce que la ligne médiane idéale du tore est déformée en une ligne fermée comportant des sections de courbures différentes tout en demeurant constamment dans un même plan.

7.  Dispositif selon la revendication 1, caractérisé en ce que le corps (1;31) est pourvu d'au moins une autre cavité (9;36,37) destinée à recevoir encore un autre capteur (9') dont la direction de mesure diffère de la direction de mesure desdits trois autres capteurs (6',7',8').

8.  Dispositif selon la revendication 2, caractérisé en ce que le corps (1) est pourvu d'une autre cavité (9) destinée à recevoir encore un autre capteur (9') dont la direction de mesure est dirigée par rapport à chacune des directions de mesure desdits trois capteurs orthogonaux entre eux (6',7',8') de façon à faire des angles égaux de même manière que la diagonale spatiale d'un cube par rapport aux arêtes de celui-ci.

9.  Dispositif selon la revendication 1, caractérisé en ce que le corps (1;31) est fait de plusieurs parties.

10. Dispositif selon les revendications 5 et 9 ou selon les revendications 6 et 9, caractérisé en ce que le corps (1) est fait de deux parties et que la réunion de ses deux parties (3,4) se fait le long d'un plan de symétrie diamétrale (5) du corps (1).

11. Utilisation du dispositif selon la revendication 1 pour la saisie de la grandeur et de la direction d'un champ magnétique dont la grandeur du champ est de l'ordre de grandeur du champ magnétique terrestre ou d'au plus deux ordres de grandeur en-dessus ou en-dessous de celui-ci.

12. Utilisation selon la revendication 11 dans une installation de recherche du nord magnétique, caractérisée en ce que le plan médian (M) du corps (1;31) est disposé de façon à faire avec le plan horizontal un angle qui dépend de la latitude géographique du lieu de mise en oeuvre du dispositif et qui vaut environ 20° dans les latitudes moyennes.

# Fig. 1

# Fig. 2

# Fig. 3